(19) **Europäisches Patentamt / European Patent Office / Office européen des brevets**

(11) **EP 4 131 282 A1**

(12) **EUROPEAN PATENT APPLICATION**
published in accordance with Art. 153(4) EPC

(43) Date of publication:
**08.02.2023 Bulletin 2023/06**

(21) Application number: **21775026.4**

(22) Date of filing: **25.03.2021**

(51) International Patent Classification (IPC):
**G16H 50/20** $^{(2018.01)}$     **G06N 20/00** $^{(2019.01)}$

(52) Cooperative Patent Classification (CPC):
**G06N 20/00; G16H 50/20**

(86) International application number:
**PCT/JP2021/012705**

(87) International publication number:
**WO 2021/193864 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2020 JP 2020054986**

(71) Applicants:
• **Hiroshima University**
  **Higashihiroshima-shi**
  **Hiroshima 739-8511 (JP)**
• **Okeios Inc.**
  **Fukuoka-shi, Fukuoka 812-0016 (JP)**
• **Nihon University**
  **Tokyo 102-8275 (JP)**
• **Data Horizon Co., Ltd.**
  **Hiroshima-shi, Hiroshima 733-0834 (JP)**

(72) Inventors:
• **KIHARA Yasuki**
  **Higashihiroshima-shi, Hiroshima 739-8511 (JP)**
• **MORIYAMA Michiko**
  **Higashihiroshima-shi, Hiroshima 739-8511 (JP)**
• **KAZAWA Kana**
  **Higashihiroshima-shi, Hiroshima 739-8511 (JP)**
• **WATANABE Hirohito**
  **Higashihiroshima-shi, Hiroshima 739-8511 (JP)**
• **NAKAMURA Takayuki**
  **Fukuoka-shi, Fukuoka 812-0016 (JP)**
• **YOSHIKAI Noriaki**
  **Tokyo 102-8275 (JP)**
• **NAGAHISA Haruhiro**
  **Hiroshima-shi, Hiroshima 733-0834 (JP)**

(74) Representative: **D Young & Co LLP**
  **120 Holborn**
  **London EC1N 2DY (GB)**

(54) **METHOD AND SYSTEM FOR DETERMINING EVENT CLASS BY AI**

(57)     Provided are novel determination techniques and systems enabling an AI process in which decisions are made from the viewpoint of allowing an expert to make a decision, and capable of providing a valid answer, even with a small amount of learning data, by adjusting errors in both the answer of the expert and the answer of AI. A system is configured to cause an AI process to be performed on subject teacher data for AI learning in which events of objects identified by identifying codes are divided into classes, and data pertaining to an object for which class determination of an event is required, and to receive class determination data obtained by the AI process. The system is configured to: cause teacher data for learning to be randomly input to, and learned by, each of a plurality of AI processes; input data pertaining to the object into each of the plurality of AI processes; receive from each AI process class determination data corresponding to each learning; and store the class determination data in a storage device. In this way, the class of an event of an object identified by each identifying code is determined on the basis of each class determination data item corresponding to the identifying code.

**(Cont. next page)**

# FIG. 6

LEARNING BY TEACHER DETERMINATION, AND COMPARISON BETWEEN
TEACHER DETERMINATION AND AI DETERMINATION

D120

ID-CLASSIFIED
FACTOR DATA

D130

| I D | CLASS |
|-----|-------|
| 0 0 0 1 | 1 |
| 0 0 0 2 | 1 |
| 0 0 0 2 | 3 |
| 0 0 0 7 | 2 |
| ⋮ | |
| 0 2 3 1 | 3 |

S140

AI(n) PROCESS

D140

| I D | TEACHER DETERMINATION | AI DETERMINATION |
|-----|-----------------------|------------------|
| 0 0 0 1 | 1 | 1 |
| 0 0 0 2 | 1 | 4 |
| 0 0 0 2 | 3 | 4 |
| 0 0 0 3 | — | 2 |
| ⋮ | | |
| 0 2 3 1 | 3 | 3 |

**Description**

TECHNICAL FIELD

The present invention relates to a method and a device for dividing events which should be classified based on an individual, a thing or information into classes from data pertaining to the individual, the thing or the information.

BACKGROUND ART

It is not uncommon that some kind of events such as ranks, grades, risks and priorities pertaining to a single individual, a thing or information are divided into classes from various kinds of data pertaining to objects such as the individual, the thing or the information.

The class division is determined based generally on a large amount of data, and reliability of the class division is increased by relying on a decision of an expert on kinds of the data pertaining to the events of the objects such as the individual, the thing or the information.

For example, in medical related matters, gravity and a degree of seriousness of a disease that each individual to be an object has as an event are generally determined by a medical worker such as a doctor who is the expert based on medical data of each individual so that the accuracy is improved.

In addition, the events of the objects such as the individual, the thing or the information change with time and the classes that the events are to be divided into also change as the events change.

Thus, if the change of the event in future based on present and past data can be estimated as the change of the class, it can contribute to better social activities such as advance risk aversion and future preparations.

In the meantime, by improvement of AI technology recent years, accuracy of a predictive determination has been improved based on various kinds of data.

However, for the AI, since a causal relation between inference for result output and a result is not clear, the utilization has been disturbed in a field where clarification of the causal relation is demanded.

Therefore, at least an AI process in which decisions are made from the viewpoint of allowing an expert to make a decision has been demanded.

Further, there are errors and slightly changing fluctuation according to an environment at the time even in a decision by an expert. In addition, when performing certain class division, the decisions are slightly different among experts as individuality of each expert, causing differences in risk division which is an answer result based on the individuality. The individuality has a point to be respected, however, when unique class division is performed by the individuality, prediction accuracy declines.

Also in an AI determination, an amount of data determined by an expert is small when compared with a large amount of data called big data, and therefore accuracy of an answer of the AI itself varies.

Thus, a novel technique of adjusting accuracy of an answer of an expert and accuracy of a determination by AI with each other while maintaining individuality of the determination of the expert has been demanded.

CITATION LIST

PATENT LITERATURE

PATENT LITERATURE 1: JP-A-2018-173814
PATENT LITERATURE 2: JP-A-2019-212146
PATENT LITERATURE 3: JP-A-2016-206950

SUMMARY OF INVENTION

TECHNICAL PROBLEM

The present invention is to provide a novel determination technique and the system from such a viewpoint, and is to provide a novel determination technique and system enabling an AI process in which decisions are made from the viewpoint of allowing an expert to make a decision, and capable of providing a valid answer, even with a small amount of learning data, by adjusting errors in both the answer of the expert and the answer of AI.

SOLUTION TO PROBLEM

The present invention provides a system configured to cause an AI (Artificial Intelligence) process to be performed on teacher data for AI learning in which events of objects identified by identifying codes are divided into classes, and data pertaining to an object for which a class determination of the event is required, and to receive class determination data obtained by the AI process.

The system is configured to: cause teacher data for learning to be randomly inputted to, and learned by, each of a plurality of AI processes; input data pertaining to the object to each of the plurality of AI processes; and receive class determination data corresponding to each learning from each AI process. Thus, the class of an event of an object identified by each identifying code is determined based on each class determination data corresponding to the identifying code.

Here, the object can be a person, the event is a severity degree pertaining to health of the person, and the severity degree is classified into a plurality of classes.

Further, the class of the event of the object identified by the identifying code can be determined to be the class to which the largest number of pieces of the class determination data obtained from each AI process belong.

Furthermore, whether or not the two or more pieces of class determination data obtained from each AI process are pertinent to a predetermined condition is determined, and the teacher data corresponding to the two or more pieces of class determination data pertinent to the predetermined condition is deleted from the entire teacher data to attain the teacher data for AI learning.

The predetermined condition may be at least based on dispersion of the two or more pieces of class determination data obtained from each AI process, and for example, it may be a condition that the dispersion of the two or more pieces of class determination data obtained from each AI process is a predetermined degree or lower.

Further, to the predetermined condition, it may be added as a condition that it is a case where the number of inconsistencies between the class indicated by the teacher data and the class indicated by the class determination data by the plurality of AI processes corresponding to the teacher data is a predetermined number or more.

Here, the class of the event of the object is the class of the event that changes according to a lapse of a time period, and can be the class of the event for which a class determination of the event to be obtained by the AI process is predicted after the lapse of the time period. For example, in medical care, utilization is available to predict the class of a severity degree that changes every fiscal year.

Further, the present invention provides a method, and the method includes steps of causing teacher data for AI learning in which events of objects identified by identifying codes stored in a storage device are divided into classes to be randomly inputted to, and learned by, each of a plurality of AI processes, and inputting data pertaining to the object for which a class determination of the event is required to each of the plurality of AI processes and receiving class determination data corresponding to each learning from each AI process by an information processor, and determining the class of the event of the object identified by each identifying code based on each class determination data corresponding to the identifying code.

BRIEF DESCRIPTION OF DRAWINGS

[FIG. 1] FIG. 1 is a drawing illustrating a configuration that an information processor which is one embodiment of the present invention is connected to a network.
[FIG. 2] FIG. 2 is a drawing illustrating a hardware configuration of the information processor in FIG. 1.
[FIG. 3] FIG 3 is a block diagram illustrating each functional configuration unit of the information processor in FIG. 1.
[FIG. 4] FIG. 4 illustrates an example of data stored in a storage unit of the information processor in FIG. 1.
[FIG. 5] FIG. 5 is a conceptual diagram illustrating a part of a process flow executed by the information processor in FIG. 1.
[FIG. 6] FIG. 6 is a conceptual diagram illustrating a part of the process flow following the process flow in FIG. 5.
[FIG. 7] FIG. 7 is a conceptual diagram illustrating a part of the process flow following the process flow in FIG. 6.
[FIG. 8] FIG. 8 is a conceptual diagram illustrating a part of the process flow following the process flow in FIG. 7.
[FIG. 9] FIG. 9 is a conceptual diagram illustrating a part of the process flow following the process flow in FIG. 8.
[FIG. 10] FIG. 10 is a conceptual diagram illustrating a present situation of a medical expense configuration examined when applying the present invention to a medical field.
[FIG. 11] FIG. 11 illustrates an object fiscal year of medical data acquired from each medical institution.

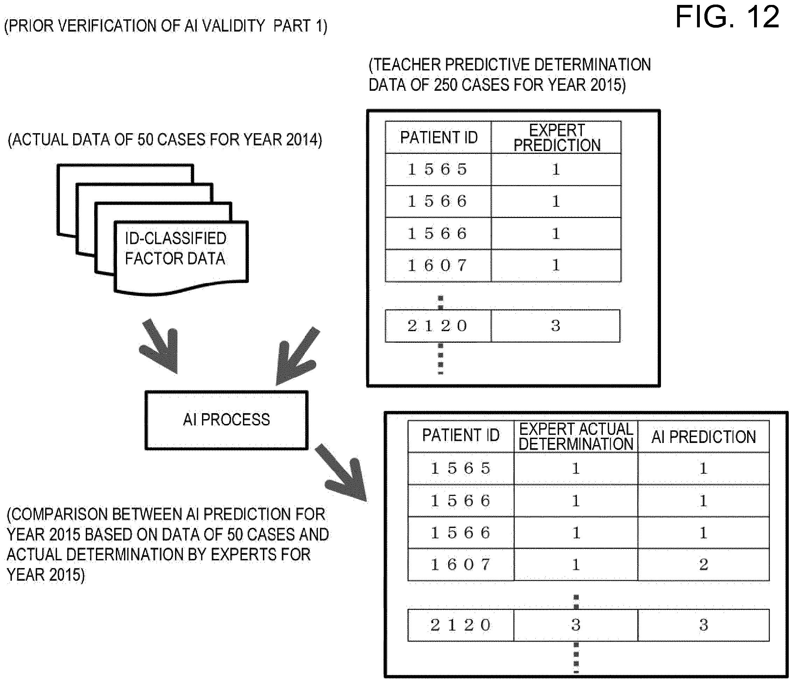

[FIG. 12] FIG. 12 illustrates an example of the process flow for verifying validity of a determination by AI.
[FIG. 13] FIG. 13 illustrates an example of a determination result by the AI based on main factors of 49 items.
[FIG. 14] FIG. 14 illustrates an example of deleting predetermined expert predictive determination data determined as abnormal from teacher data by a medical expert predictive determination and a plurality of AI determinations.
[FIG. 15] FIG. 15 illustrates an example of an AI predictive determination by adjusted teacher data.

DESCRIPTION OF EMBODIMENTS

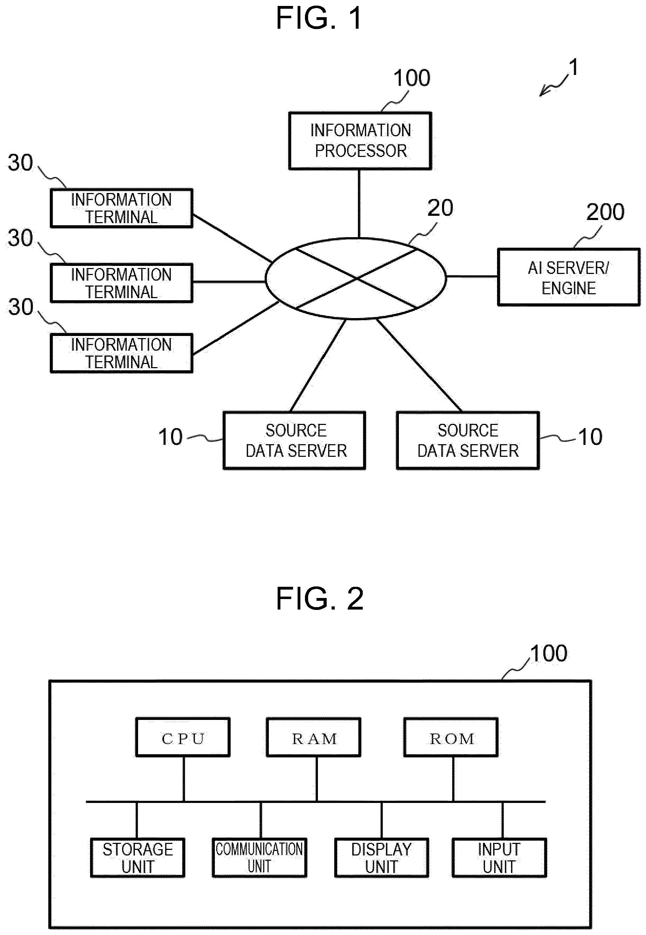

FIG. 1 illustrates a system 1 according to one embodiment of the present invention. As one embodiment, an information processor 100 is connected to a network 20. A user can access the information processor 100 via an information terminal 30 and receive a service provided by the information processor 100. Accessing is performed via a wired or wireless network 20 such as the Internet, a private line or an indoor line. The number of accessible information terminals is not limited in particular, and appropriate utilization is possible according to a purpose of utilization. The information processor 100 is configured to receive input data from the information terminal 30 via the network 20 and provide a corresponding service.

A source data server 10 is configured to provide the information processor 100 with required data according to service contents of the information processor 100 via the network 20. When needed, mutual authentication and agreement formation for data handling may be performed beforehand among the information processor 100, the source data server and the information terminal 30.

An AI server or a server engine 200 can be configured by combining hardware such as a CPU, a multi-processor mounted with a plurality of processor cores, GPUs (Graphics Processing Units), DSPs (Digital Signal Processors), and FPGAs (Field-Programmable Gate Arrays), and may be configured by algorithm or a learned model or the like which realizes a machine learning function, a language analysis function and a voice recognition function further or the like. A quantum processor can be also combined, and the machine learning function may be achieved by, for example, a neural network including deep learning or reinforcement learning. Note that the server can be configured by one or two or more servers, and individual components such as an AI engine unit and a storage unit may be distributedly arranged.

Next, a hardware configuration of the information processor 100 will be explained with reference to FIG. 2.

The information processor 100 according to an example of the present embodiment includes a CPU (Central Processing Unit), a RAM (Random Access Memory), a ROM (Read Only Memory), a storage unit, a communication unit including a network I/F (Interface), a display unit and an input unit or the like. As needed, other hardware configuration may be included.

The CPU is an arithmetic unit which realizes control and functions of the information processor 100 by reading a program and data stored in the ROM and the storage unit or the like and executing a process while storing required data on the RAM.

The storage unit is a storage device such as an HDD (Hard Disk Drive) and an SSD (Solid State Drive), for example, for storing an OS (Operating System) and various kinds of application programs or the like.

The communication unit has a communication interface function for providing an interface of the information processor 100 and the network 20 and performing information communication with an external device such as the information terminal 30, the source data server 10 and the AI server 200 connected to the network 20.

The display unit is a display device such as a display, and displays a processing result by the information processor 100 to the user. The input unit can be an input device such as a keyboard, a mouse, a camera and a microphone, etc..

A bus transmits address signals, data signals and various kinds of control signals or the like in order to connect individual configuration units inside the information processor 100.

Note that the information processor 100 is not limited to the configuration described above and may be distributed and realized by separate computers, individual components of the information processor 100 may be distributed and integrally function, or individual elements configuring the information processor 100 may be single or formed of a plurality of combinations. Further, depending on demanded service contents, a required application may be distributed between the information terminal 30 and the information processor 100 to realize the service in cooperation.

In addition, the system is not limited to the hardware configuration indicated in the present embodiment, and may be in any form as long as the present invention can be implemented. Further, an external AI process function provided by nonofficial or the like may be utilized.

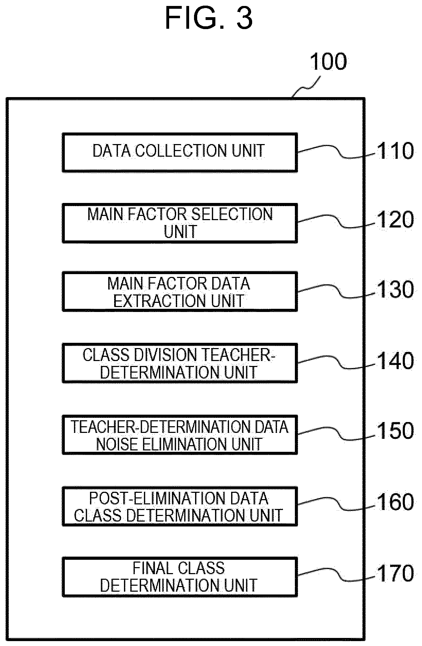

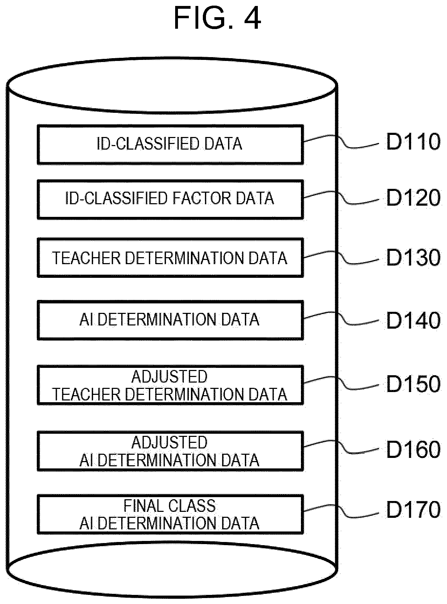

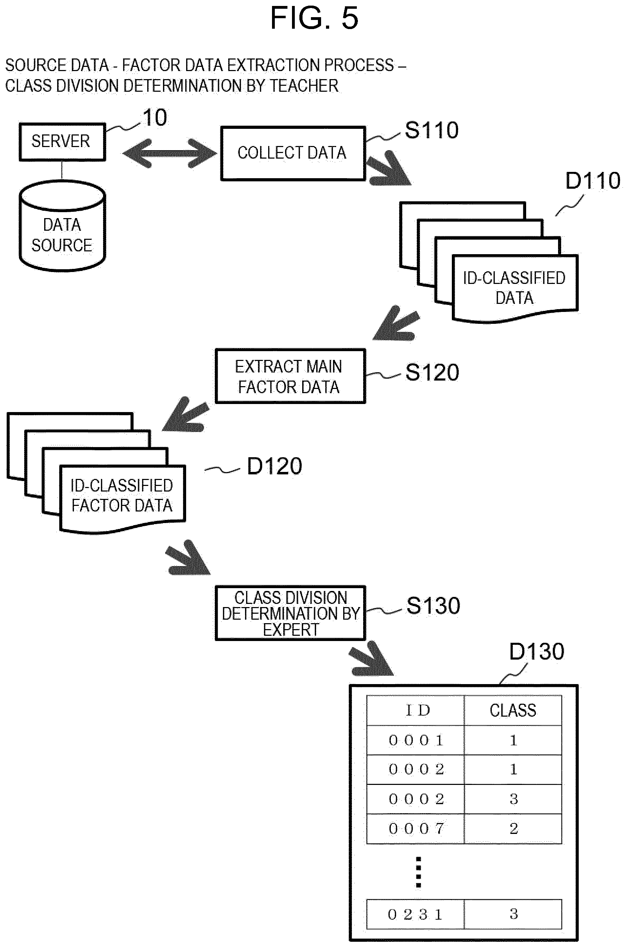

FIG. 3 illustrates an example of the functions that the information processor 100 has. FIG. 4 illustrates various kinds of data stored in the storage unit of the information processor 100, and FIG. 5 to FIG. 9 illustrate a series of main process flows based on the embodiment of the present invention.

A data collection unit 110 collects data to be required when determining a class of an object event (step S110 in FIG. 5) and stores the data as ID-classified data D 110 which can be managed by identification data (ID).

A main factor selection unit 120 allows the user utilizing the method and system of the present invention to select a factor to be utilized when determining a class. Instead of the user, the system 1 may automatically make a determination and select an effective factor.

A main factor data extraction unit 130 extracts, according to the factor selected by the main factor selection unit 120, data for each ID related to the factor (hereinafter, also called "ID-classified factor data" or simply "factor data") from the data collected in the data collection unit 110, and stores the data as ID-classified factor data D120 (step S120).

Note that the factor may be selected beforehand, and the factor data to be required may be collected from the source data server 10 based on the selected factor.

The identification data is the data capable of uniquely identifying the object such as an individual person, a company, a thing and information, and the collected data and the extracted ID-classified data D110 include various kinds of data which can be linked or associated to the identification data.

For example, the identification data is, for an individual person, a name itself or an identifying code capable of specifying the name (for example, a patient ID at the time of attending a hospital, an account number at the time of a bank transaction, and an ID used when purchasing merchandise or the like by online shopping or the like). Needless to say, the identification data may be many IDs or accounts or the like as long as the individual person or the company or the like can be specified, and the data linked to them may be generally collected and integrated.

When the various kinds of data that can be linked to the identification data can be utilized to divide some kind of events pertaining to the object such as an individual, a company, a thing and information into classes from the data, the kind does not matter.

For example, the object is a company, the events can be divided into classes as soundness of each company, and the various kinds of data may be any information related to the soundness of the company.

A class division teacher determination unit 140 uses some ID-classified factor data of the ID-classified factor data D120 extracted from the ID-classified data D 110 as class determination (step S130). Alternatively, the ID-classified factor data may be extracted directly from the ID-classified data D 11 0 and used for the class determination. In order to enable reliable class determination, it is preferable that the determination is performed by an expert or an intellectual or the like (hereinafter, simply called an "expert") who is well informed about business practice related to the data.

The expert of the business practice may input a class determination result to the information processor 100 via the information terminal 30. For that, the information processor 100 displays information of the extracted factor data to the expert via the information terminal 30, and the expert can input the class determination result to the information terminal 30 based on his/her own experiences and knowledge on the basis of the displayed data, and store the class determination result in the information processor 100.

It is preferable that the plurality of experts determine the classes, and the information processor 100 stores the class determination result by each of the plurality of experts in the information processor 100.

Accordingly, step S130 performed by the class division teacher determination unit 140 may be realized as a simple interface application capable of providing the factor data from the information processor 100 to the information terminal 30 so that the expert can determine the classes and providing determination data from the information terminal 30 to the information processor 100, or may be realized as a simple application by which the expert can input the determination data to the information processor 100. The classes may be determined redundantly and uniquely by different experts based on same information based on the same identifying code.

Since the class determination result is inputted to the AI and used as data for learning, the class determination result can be called teacher determination data or teacher data D130 as a generalized name here.

The teacher determination data D130 may be future predictable class discrimination data in addition to the present class discrimination based on the factor data. Future prediction includes prediction of the next fiscal year and/or prediction of the next month, and the future period is not limited in particular. In addition, the future period may be appropriately set according to a period to which the collected data belongs.

A teacher determination data noise elimination unit 150 eliminates data with which the class determination result is handled as abnormal, such as unique data or abnormal data from the teacher determination data inputted for learning, from the entire teacher determination data as the data with noise for example.

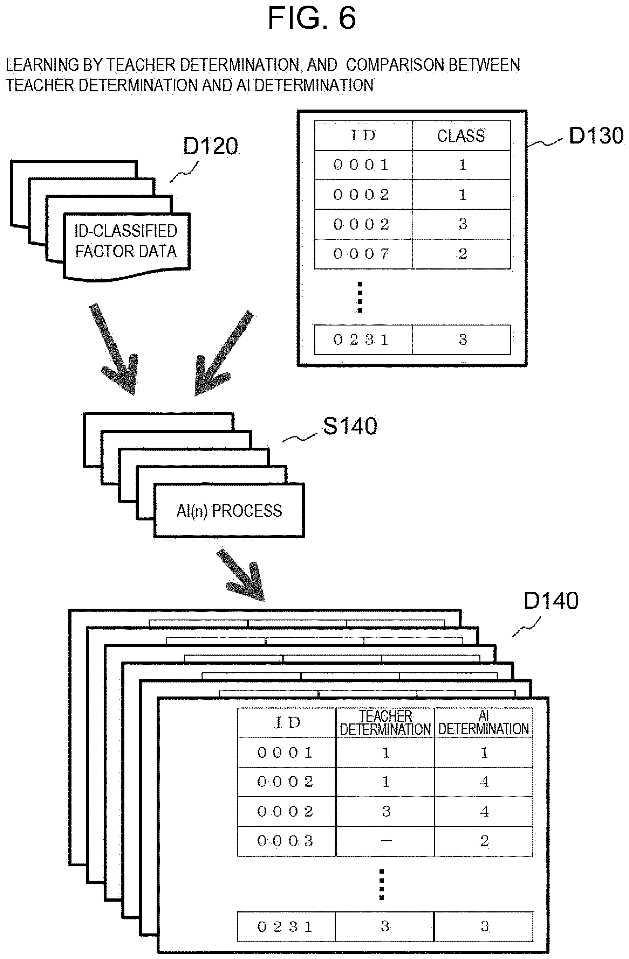

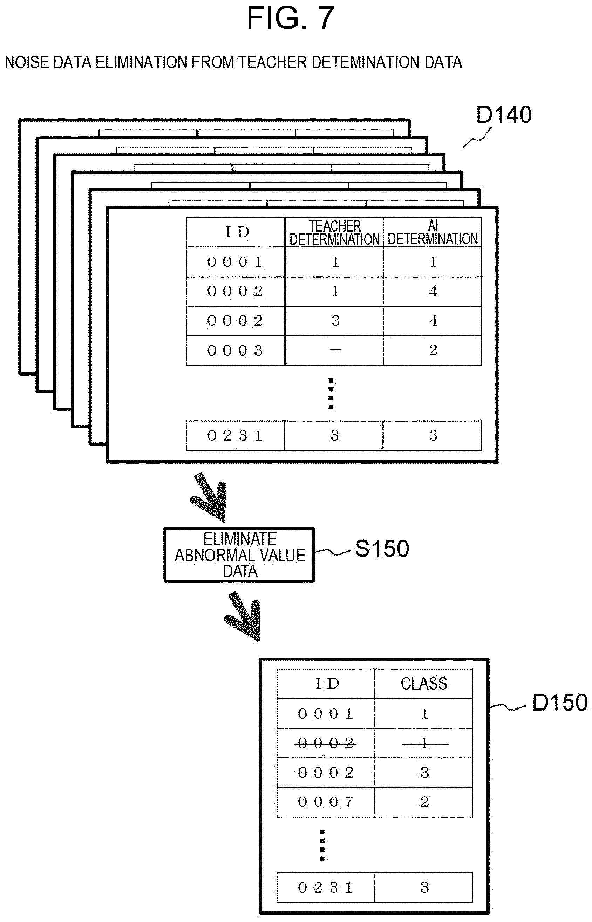

One embodiment of the elimination method performed by the teacher determination data noise elimination unit 150 will be explained with reference to FIG. 6 and FIG. 7.

First, the teacher determination data D130 is inputted to an AI process of the AI server 200 or the like, and learning according to the contents of the teacher determination data D130 is performed. In addition, the ID-classified factor data D120 is inputted to the AI process as a class prediction object, and outputted to the information processor 100 as AI determination data D140 accompanied by the class determination according to the learning (step S140).

Here, for the ID-classified factor data used for the determination by the expert as the teacher determination data D130, the one partially extracted from the ID-classified factor data D120 can be utilized as illustrated in FIG. 5, however, the main factor data extraction unit 130 may collect the ID-classified factor data D120 and the ID-classified factor data to be used for the determination by the expert separately from an external server. That is, when the ID-classified factor data D120 to be used for the determination by the AI and the ID-classified factor data to be supplied to the AI for learning are to be acquired and prepared through what route is not limited in particular.

The AI determination data D140 can be accompanied by the class by teacher determination for each individual identifying code (ID), that is, the class determined by the expert, and the class determined by the AI.

It is preferable to perform the learning to the AI and the determination by the AI by a plurality of AI processes. In addition, it is preferable to input the data to the plurality of AIs randomly. The teacher determination data D130 and the ID-classified factor data D120 that are randomly inputted are individually processed by the plurality of AIs (step S140), and individual AI determination data is received and stored in the information processor 100 as output of each AI. Then, from the AI determination data, two or more pieces of the AI determination data D140 according to the identifying codes are created.

Next, for each identifying code, from the data of the class division by the teacher determination and the class division by each AI, teacher determination data determined as an abnormal value is deleted. FIG. 7 illustrates an example of deleting the teacher determination data in which the class is determined as 1 for the ID of 0002 from the determination (step S150 in FIG. 7).

For the determination of the abnormal value, for example, in the case where a predetermined difference or distance is generated between the class by the AI determination and the class by the teacher determination, the teacher determination data is processed as the noise and removed from the entire teacher determination data (step S150).

For example, when the classes are divided into 1-4 and numbered, in the case where there is the difference of a predetermined value, for example, 3 or larger between the class by the AI determination and the class by the teacher determination, it can be determined as the abnormal value. It is needless to say that a technique of defining the abnormal class determination is not limited thereto and other appropriate determination techniques can be applied. In such a manner, by managing the class as a number, the information processor 100 can perform execution as a mathematical computational process.

In the case of performing prediction by the plurality of AIs, Theory of Collective Intelligence may be utilized. The theory of collective intelligence can be expressed by a following equation.

$$\text{Group error=average individual error-variance}$$

The group error is a difference between an average of values estimated by members of a certain group and a correct answer. The average individual error is an average value of errors of the individual members, and the variance is dispersion of estimated values of the individual members.

Mathematically, it can be expressed that,

when an estimated value of a member i is defined as Xi (i=1, 2, ..., N), and
a group estimated value is A and a true value is R,

$$\text{Group error}=(A\text{-}R)^2$$

$$A=\{X(1)+X(2)+...+X(N)\}/N$$

$$\text{Average individual error}=\{(X(1)\text{-}R\}^2+(X(2)\text{-}R)^2+..+(X(N)\text{-}R)^2\}/N$$

$$\text{Variance}=\{(X(1)\text{-}A)^2+(X(2)\text{-}A)^2+..+(X(N)\text{-}A)^2\}/N.$$

The theory of collective intelligence indicates that a presumption error (first term) of an individual in a group is offset by diversity (second term) and presumption close to the correct answer can be performed as the group.

When it is applied to the present invention, when AI(1)-AI(n) (n is an integer) which are AI servers or AI engines are defined as individual members and ranks predictively determined by the individual members are defined as estimated values, determination results of the plurality of AIs should indicate a distribution centering on the correct answer, and according to the theory of collective intelligence, it means that, when the variance of the determination results of the individual members is larger, accuracy that the answer indicated by the average of the group becomes the correct answer increases compared to the determination result of each AI.

In order to delete the data of abnormal class determination in the teacher data and reduce the entire error, it can be a condition that the error of the AI is small and the variance is large based on the theory of collective intelligence, the data to be deleted can be narrowed down to the data of a large error and a small variance or standard deviation.

Accordingly, in the present embodiment, the case where the dispersion of the rank determined by the AI is within a predetermined range and the standard deviation is a certain threshold or smaller for example is defined as one condition, and the teacher data corresponding to the pertinent AI determination is deleted from the entire teacher data. Alternatively, or in addition to the condition, the teacher data may be defined as the abnormal value on condition that the case where a predetermined number or more of the AIs cause the difference from the class of the teacher data between the class prediction of the plurality of AIs and the class of the teacher data, and the teacher data may be deleted from the entire teacher data.

For example, in the case where the number of the AIs is 10, the standard deviation of the determination of the AIs with each other is 0.5 or smaller and the number of the AIs where the determination of the AI and the teacher data are inconsistent is 7 or larger, the teacher data may be defined to have the abnormal value due to unneglectable fluctuation or deviation and be eliminated.

Note that a term "abnormal" or "abnormal value" used here is conveniently used in order to eliminate the teacher determination data under a specified condition, and it should be understood that the term "abnormal" is to generally indicate the object to eliminate the teacher data determined as not suitable for the AI process.

As illustrated in FIG. 7, the teacher determination data from which the teacher determination data determined as being abnormal is eliminated is stored as adjusted teacher determination data D150.

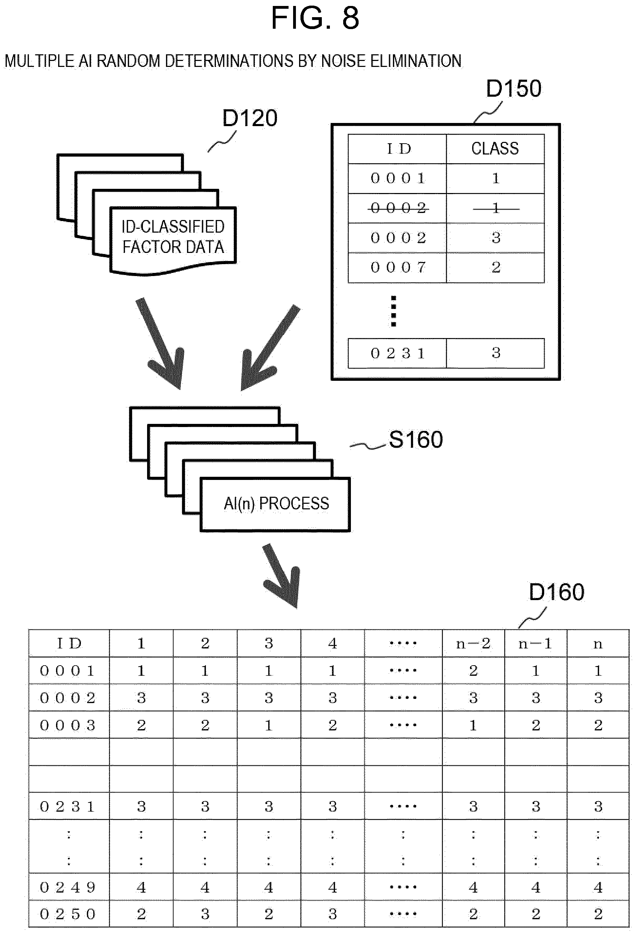

Referring to FIG. 8, a post-elimination data class determination unit 160 inputs the adjusted teacher determination data D150 which includes two or more pieces of factor data related to the plurality of identifying codes and from which abnormal data is eliminated to the AI server 200 described above for utilization as learning data. Further, a group of the ID-classified factor data D120 related to one, two or more identifying codes to be divided into classes are also inputted to the AI server 200. By the AI process, based on the group of the ID-classified factor data, the AI determination of the class division corresponding to each identifying code is executed (step S160). The post-elimination data class determination unit 160 receives the determination result by the AI and stores it as AI determination data D160.

At the time, it is preferable to also input the adjusted teacher determination data D150 and the ID-classified factor data D120 to the plurality of AI(1)-AI(n) and perform the predictive determination. In addition, it is preferable to input the data to the plurality of AIs randomly. The adjusted teacher determination data D150 and the ID-classified factor data D120 that are inputted randomly are individually processed by the plurality of AIs and adjusted individual AI determination data is stored in the information processor 100 as the output of each AI. Then, from the AI determination data, final class AI determination data D160 according to the identifying codes is created.

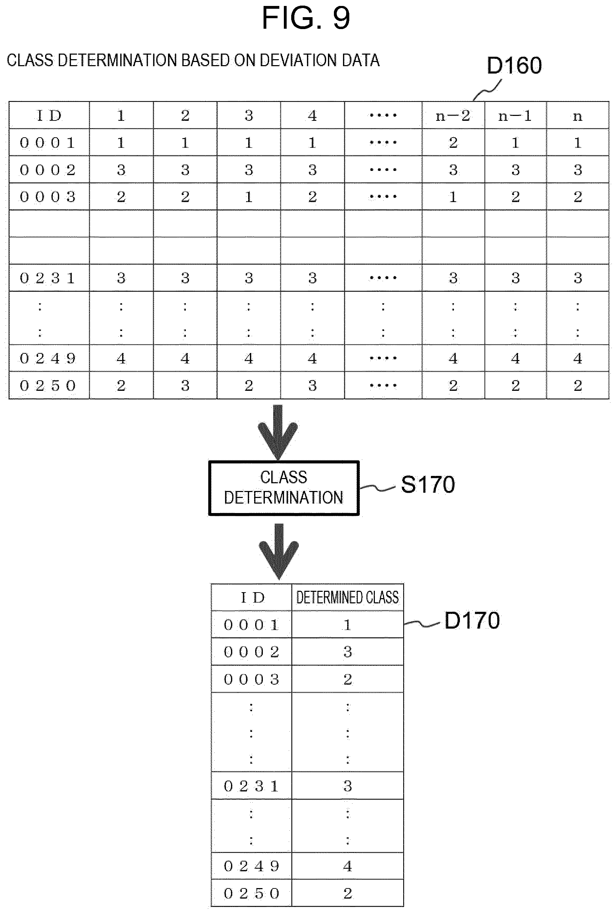

As illustrated in FIG. 9, the final class determination is performed by a final class determination unit 170 (step S170). Final determined class data D 170 is created from the class determination of the AI(1)-AI(n) for each identifying code listed in the final class AI determination data D 160. The class determination standard may be determined from a midmean of the classes predicted by the AI(1)-AI(n), the most common class may be determined as the final class, or there may be other appropriate determination methods.

Next, examples of applying the preset invention to a medical field will be explained.

EXAMPLE 1

(Prior confirmation of AI validity Part 1)

To implement the present invention, whether or not an AI utilization method by a basic idea of the present invention is effective was confirmed in advance.

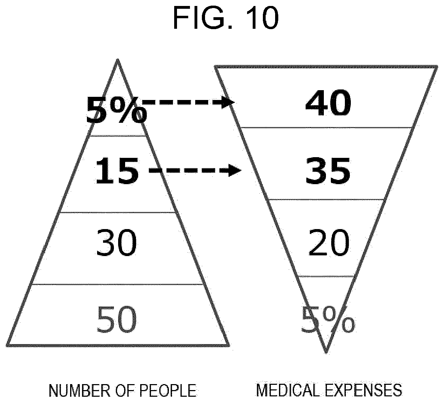

In FIG. 10, conditions of people who had a health checkup is divided into four layers as classes, and an outline of a relation between the number of people in each layer and medical expenses is illustrated.

The classes are numbered as a class 1, a class 2, a class 3 and a class 4 from a bottom layer, in which the class 1 is the condition of expecting continuous health promotion, the class 2 is the condition of a light level, the class 3 is the condition of a middle level and the class 4 is the condition of a severe level.

The four layers are utilizable when measuring a risk of a disease to people and recognizable as levels of severity, and it can be determined that the severity level is highest for the top level and the severity level is almost zero for the bottom layer. Thus, the class division is risk division and the present invention can be utilized as risk management.

When performing the class division, ratios of the numbers of people in the individual layers to the number of the entire people form an almost pyramid shape of 50%, 30%, 15% and 5% from the bottom, though it depends on how they are divided. On the other hand, the medical expenses of the individual layers to the entire medical expenses forms an inverse pyramid distribution of 35%, 20% and 5% toward the bottom with 40% in the class 4 as the top layer.

Accordingly, when the generation of the class 4 and the class 3 in which the number of people is 5% and 15% is predicted and prevented, more appropriate care for people with a possibility of being in the severe level is made possible, and the entire medical expenses can be reduced.

Then, first, in order to verify the validity of the present invention in advance, health checkup and medical data (hereinafter, simply called "medical data") for 250 people was acquired from medical institutions, and doctors engaged in medical treatment and other experts (simply called "experts") were asked to visually observe the medical data of the year 2014 and predict a risk level of severity of each person for the year 2015 which is the next fiscal year.

Then, the risk numbers were divided into numbers 1-4, and the medical data of each person accompanied by the risk numbers 1-4 was inputted to the AI as the learning data. Further, to the learned AI, the medical data of 50 people out of 250 people for the year 2014 was inputted and the severity risk for the year 2015 was predicted.

As a result, out of the 50 people, eight cases (people) were different between the prediction for the year 2015 by the experts and the prediction for the year 2015 by the AI.

Further, when the severity level of the year was determined by the experts from the medical data for the year 2015 of the 50 people and the actual result was compared with both pieces of prediction data by the experts and the AI, out of the eight different cases, the severity level of two cases predicted by the AI and the actual result were consistent.

From that, though it is the data of a small scale, the possibility of the AI validity was confirmed.

EXAMPLE 2

(Prior confirmation of AI validity Part 2)

While the data obtained from the medical institutions include many kinds, when items which are factors affecting individual diseases obtainable by individual health guidance and which can be acquired from medical prescription data and health checkup data are narrowed down to a fixed number and sorted out as main factors, the prediction becomes possible broadly even for general people to be medically treated, a utilization range is expanded and prediction accuracy is also improved.

From such a viewpoint, though the process will not be described in details here, factor data of 49 items was to be used.

First, the information processor 100 collected the data of "medical treatment information statement", "nursing care benefit statement", "special health checkup result" and "insured master" from the data servers 10 (FIG. 1) of various kinds of medical institutions to configure ID-classified data D110, and the ID-classified factor data D120 of 2699 cases including the factor data for the year 2014 of the 49 selected items was created and stored.

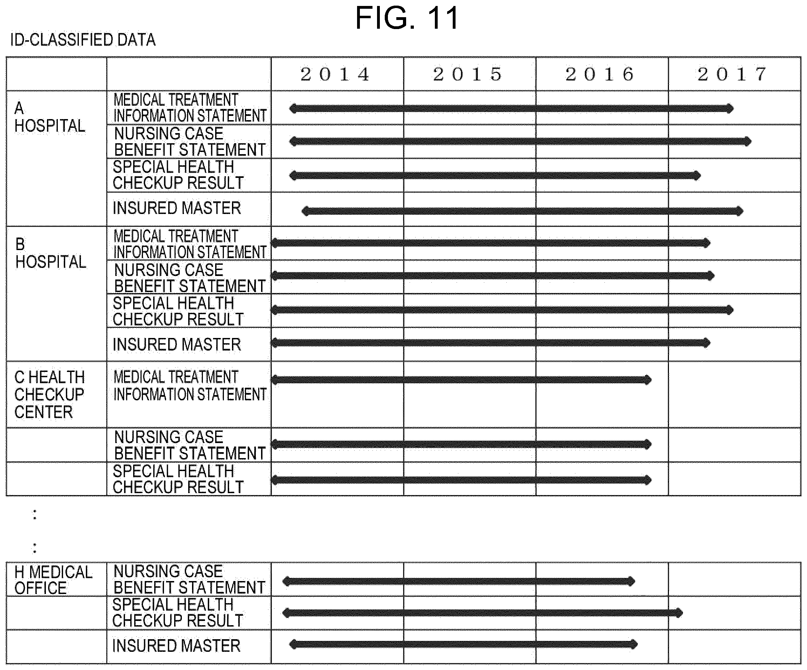

Note that FIG. 11 illustrates the corresponding years of the data collected from the individual medical institutions.

Next, the ID-classified factor data D120 of 2699 cases stored in the information processor 100 was read, the contents of the ID-classified factor data D 120 was displayed via the information terminal 30 utilized by 25 experts, and clinical inference by the experts based on each data of 49 items for the year 2014 was performed.

Risk prediction for the year 2015 obtained by the clinical inference was inputted to the information terminal 30, and stored in the information processor 100 as the teacher determination data D130 for each ID of 2699 cases including the risk prediction.

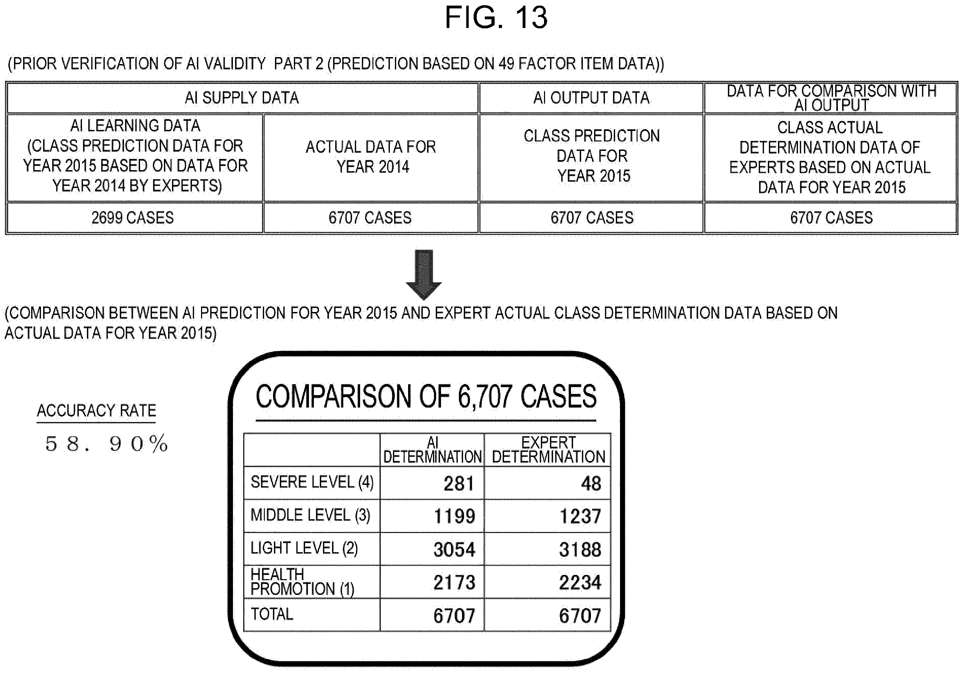

Further, as illustrated in FIG. 13, the information processor 100 inputted the teacher determination data D130 of the 2699 cases to the AI as the data for AI learning to be learned.

In addition, as the data to be used for the risk prediction for the year 2015, the ID-classified factor data D120 of 6707 cases, which is actual data of the year 2014 collected and extracted by the information processor 100, was inputted to the AI server 200.

As a result, the information processor 100 received the prediction data (class prediction data for the year 2015) of 6707 cases predicted by the AI server 200 from the AI server 200.

In order to confirm accuracy of results predicted by the AI server 200, by the experts, the risk division was performed by the clinical inference based on the actual data of 6707 cases of the year 2015, and the result (actual class determination by teacher based on the actual data of the year 2015) and the AI prediction (the class prediction for the year 2015) were compared.

For the result, a class consistent ratio was 58.90%, and the ratio of each class was as illustrated in FIG. 13.

From above, it was understood that it was possible to predict the severity level for the next year from the data of a certain year, however, the prediction with higher accuracy was also desired.

EXAMPLE 3

(Elimination of unrequired fluctuation and deviation by multiple AI determinations and improvement of prediction)

As a result of investigating the contents of the teacher data, it was recognized that the severity level prediction varied and the severity level prediction was way different from the others depending on the experts and that also affected the accuracy of the AI prediction as the fluctuation of the teacher data.

Accordingly, as explained in the flow in FIG. 6-FIG. 9, it was decided to utilize the plurality of AI prediction results by the plurality of AIs, perform comparison with the expert prediction, and perform final severity prediction from the AI prediction again based on the data from which the severity prediction determined as being abnormal by the comparison was eliminated.

The teacher determination data D130 from the ID-classified factor data D120 of 2699 cases and the ID-classified factor data D120 of 6707 cases collected and extracted by the information processor 100, which are explained in EXAMPLE 2, were utilized, input was performed from the information processor 100 to the plurality of AIs in a random order of the data within the teacher determination data D130, and the AI prediction was performed. The severity levels by the plurality of AIs obtained as a result were compared with the corresponding predicted severity levels by the experts according to the IDs.

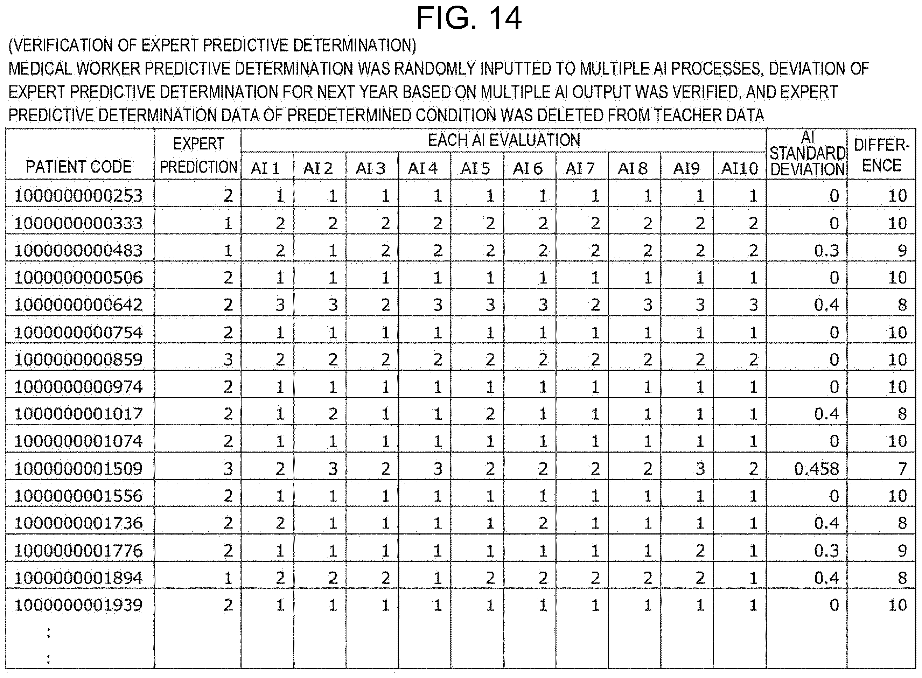

For the comparison, in addition to the comparison between expert determination data and each determination data of the AI(1)-AI(n), the determinations among the AI(1)-AI(n) were also performed, and as illustrated as a list in FIG. 14, based on the already explained theory of collective intelligence, the expert determination data, for which it is the case where the number of inconsistencies between the expert determination data and the AI determination data is 7 or larger and the deviation of the determinations between the AIs is smaller than 0.5 as the standard deviation, was deleted.

Note that the number illustrated here is an example and the determination as the abnormal value is possible by an arbitrary value.

In this way, according to the step illustrated in FIG. 7, the teacher data determined to have the abnormal value was deleted from the entire teacher data of 2699 cases, and new teacher data D150 of 2228 cases was created.

From the new teacher data D150 and the ID-classified factor data of 6707 cases of the year 2014, as in the step illustrated in FIG. 8, the information processor 100 made the plurality of AIs perform learning randomly and receives the predicted determination result from the Ai server.

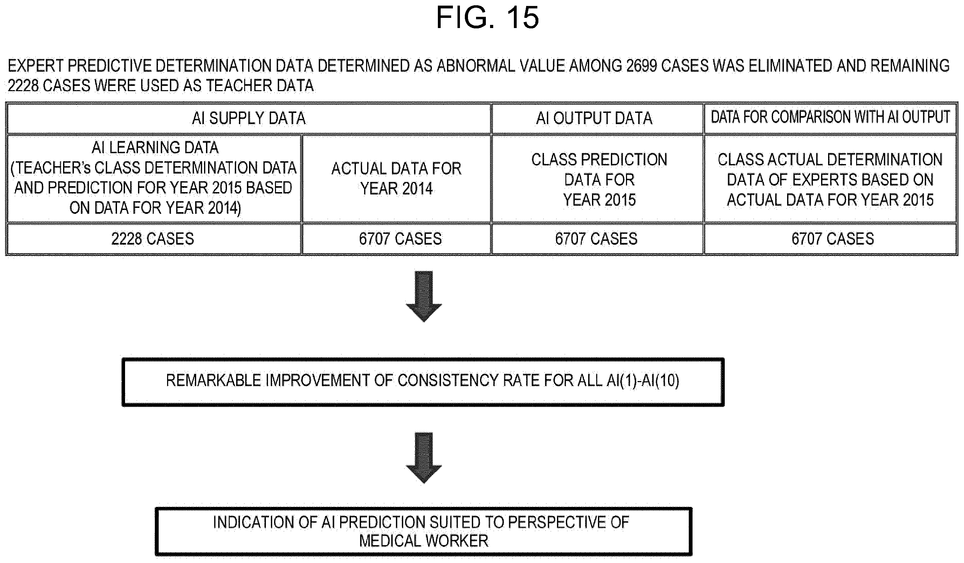

As illustrated in FIG. 15, as a result of comparing the class prediction data for the year 2015 of 6707 cases outputted from the AI server and the class actual determination by the experts based on the actual data of the year 2015, remarkable improvement of 83% or higher was observed for all the AI(1)-AI(n) (n=10).

This indicates that the AI prediction suited to a perspective of general people engaged in the medical treatment is possible, and indicates a reason that a wide application in a medical range is possible.

In addition, since the explained four layers are also utilizable when measuring a risk of a disease to people, the class division corresponds to the risk division, and each layer can be recognized also as a risk layer. Thus, it can be understood that the AI prediction based on the class division by the present invention is effective also for the risk management.

While the embodiment of the present invention has been explained as above, various alternative examples, corrections or modifications are possible for persons skilled in art based on the explanation above, and the present invention includes the various alternative examples, correction or modifications described above without deviating from the gist.

REFERENCE SIGNS LIST

10      Source data server

20      Network

30      Information terminal

100     Information processor

200     AI server or server engine

**Claims**

1. A system for causing an AI (Artificial Intelligence) process to be performed on teacher data for AI learning in which events of objects identified by identifying codes are divided into classes, and data pertaining to an object for which a class determination of the event is required, and receiving class determination data obtained by the AI process, the system being configured to

cause the teacher data for learning to be randomly inputted to, and learned by, each of a plurality of AI processes; input the data pertaining to the object to each of the plurality of AI processes; and receive the class determination data corresponding to each learning from each AI process,

wherein the class of the event of the object identified by each identifying code is thus determined based on each class determination data corresponding to the identifying code.

2. The system according to claim 1, wherein the teacher data corresponding to two or more pieces of class determination data pertinent to a predetermined condition is deleted from the entire teacher data to attain teacher data for AI learning.

3. The system according to claim 2, wherein the predetermined condition is at least based on dispersion of the two or more pieces of class determination data obtained from each AI process, or at least based on whether or not the dispersion of the two or more pieces of class determination data obtained from each AI process is a predetermined degree or lower.

4. The system according to claim 3, wherein, further, for the predetermined condition, a condition is that it is a case where the number of inconsistencies between the class indicated by the teacher data and the class indicated by the class determination data by the plurality of AI processes corresponding to the teacher data is a predetermined number or more.

5. The system according to claim 1, wherein the class of the event of the object is the class of the event that changes according to a lapse of a time period, and is the class of the event for which a class determination of the event to be obtained by the AI process is predicted after the lapse of the time period.

6. The system according to claim 1 or 2, wherein the class of the event of the object identified by the identifying code is determined to be the class to which the largest number of the pieces of class determination data obtained from each AI process belong.

7. The system according to claim 1, wherein the object is a person and the event is a severity degree of the person.

8. A method comprising:

inputting randomly, by an information processor, training data for AI learning in which events of objects identified by identifying codes stored in a storage device are divided into classes to each of a plurality of AI processes, and inputting data pertaining to the object for which a class determination of the event is required to each of the plurality of AI processes, and receiving class determination data corresponding to each learning from each AI process by the information processor,
wherein the class of the event of the object identified by each identifying code is determined based on each class determination data corresponding to the identifying code.

9. The method according to claim 8, wherein the teacher data corresponding to two or more pieces of class determination data pertinent to a predetermined condition is deleted from the entire teacher data to attain the teacher data for AI learning.

10. The method according to claim 9, wherein the predetermined condition is at least based on dispersion of the two or more pieces of class determination data obtained from each AI process, or at least based on whether or not the dispersion of the two or more pieces of class determination data obtained from each AI process is a predetermined degree or lower.

11. The method according to claim 10, wherein, further, for the predetermined condition, a condition is that it is a case where the number of inconsistencies between the class indicated by the teacher data and the class indicated by the class determination data by the plurality of AI processes corresponding to the teacher data is a predetermined number or more.

12. The method according to claim 8, wherein the class of the event of the object is the class of the event that changes according to a lapse of a time period, and is the class of the event for which a class determination of the event to be obtained by the AI process is predicted after the lapse of the time period.

13. The method according to claim 8 or 9, wherein the class of the event of the object identified by the identifying code is determined to be the class to which the largest number of pieces of the class determination data obtained from each AI process belong.

**14.** The method according to claim 8, wherein the object is a person and the event is a severity degree of the person.

# FIG. 1

# FIG. 2

# FIG. 3

100

| |
|---|
| DATA COLLECTION UNIT ——110 |
| MAIN FACTOR SELECTION UNIT ——120 |
| MAIN FACTOR DATA EXTRACTION UNIT ——130 |
| CLASS DIVISION TEACHER-DETERMINATION UNIT ——140 |
| TEACHER-DETERMINATION DATA NOISE ELIMINATION UNIT ——150 |
| POST-ELIMINATION DATA CLASS DETERMINATION UNIT ——160 |
| FINAL CLASS DETERMINATION UNIT ——170 |

# FIG. 4

ID-CLASSIFIED DATA — D110

ID-CLASSIFIED FACTOR DATA — D120

TEACHER DETERMINATION DATA — D130

AI DETERMINATION DATA — D140

ADJUSTED
TEACHER DETERMINATION DATA — D150

ADJUSTED
AI DETERMINATION DATA — D160

FINAL CLASS
AI DETERMINATION DATA — D170

# FIG. 5

SOURCE DATA - FACTOR DATA EXTRACTION PROCESS –
CLASS DIVISION DETERMINATION BY TEACHER

| I D | CLASS |
|------|-------|
| 0 0 0 1 | 1 |
| 0 0 0 2 | 1 |
| 0 0 0 2 | 3 |
| 0 0 0 7 | 2 |
| ⋮ | |
| 0 2 3 1 | 3 |

# FIG. 6

LEARNING BY TEACHER DETERMINATION, AND COMPARISON BETWEEN
TEACHER DETERMINATION AND AI DETERMINATION

D120

ID-CLASSIFIED
FACTOR DATA

D130

| I D | CLASS |
|------|-------|
| 0 0 0 1 | 1 |
| 0 0 0 2 | 1 |
| 0 0 0 2 | 3 |
| 0 0 0 7 | 2 |
| ⋮ | |
| 0 2 3 1 | 3 |

S140

AI(n) PROCESS

D140

| I D | TEACHER DETERMINATION | AI DETERMINATION |
|------|------------------------|-------------------|
| 0 0 0 1 | 1 | 1 |
| 0 0 0 2 | 1 | 4 |
| 0 0 0 2 | 3 | 4 |
| 0 0 0 3 | – | 2 |
| ⋮ | | |
| 0 2 3 1 | 3 | 3 |

# FIG. 7

NOISE DATA ELIMINATION FROM TEACHER DETEMINATION DATA

D140

| ID | TEACHER DETERMINATION | AI DETERMINATION |
|---|---|---|
| 0 0 0 1 | 1 | 1 |
| 0 0 0 2 | 1 | 4 |
| 0 0 0 2 | 3 | 4 |
| 0 0 0 3 | — | 2 |
| ⋮ | | |
| 0 2 3 1 | 3 | 3 |

ELIMINATE ABNORMAL VALUE DATA — S150

| ID | CLASS |
|---|---|
| 0 0 0 1 | 1 |
| ~~0 0 0 2~~ | ~~1~~ |
| 0 0 0 2 | 3 |
| 0 0 0 7 | 2 |
| ⋮ | |
| 0 2 3 1 | 3 |

D150

# FIG. 8

MULTIPLE AI RANDOM DETERMINATIONS BY NOISE ELIMINATION

D150

D120

ID-CLASSIFIED
FACTOR DATA

| I D | CLASS |
|------|-------|
| 0 0 0 1 | 1 |
| ~~0 0 0 2~~ | ~~1~~ |
| 0 0 0 2 | 3 |
| 0 0 0 7 | 2 |
| ⋮ | |
| 0 2 3 1 | 3 |

S160

AI(n) PROCESS

D160

| I D | 1 | 2 | 3 | 4 | ···· | n−2 | n−1 | n |
|------|---|---|---|---|------|-----|-----|---|
| 0 0 0 1 | 1 | 1 | 1 | 1 | ···· | 2 | 1 | 1 |
| 0 0 0 2 | 3 | 3 | 3 | 3 | ···· | 3 | 3 | 3 |
| 0 0 0 3 | 2 | 2 | 1 | 2 | ···· | 1 | 2 | 2 |
| | | | | | | | | |
| | | | | | | | | |
| 0 2 3 1 | 3 | 3 | 3 | 3 | ···· | 3 | 3 | 3 |
| ⋮ | ⋮ | ⋮ | ⋮ | ⋮ | ⋮ | ⋮ | ⋮ | ⋮ |
| ⋮ | ⋮ | ⋮ | ⋮ | ⋮ | ⋮ | ⋮ | ⋮ | ⋮ |
| 0 2 4 9 | 4 | 4 | 4 | 4 | ···· | 4 | 4 | 4 |
| 0 2 5 0 | 2 | 3 | 2 | 3 | ···· | 2 | 2 | 2 |

# FIG. 9

CLASS DETERMINATION BASED ON DEVIATION DATA

D160

| ID | 1 | 2 | 3 | 4 | ···· | n−2 | n−1 | n |
|---|---|---|---|---|---|---|---|---|
| 0001 | 1 | 1 | 1 | 1 | ···· | 2 | 1 | 1 |
| 0002 | 3 | 3 | 3 | 3 | ···· | 3 | 3 | 3 |
| 0003 | 2 | 2 | 1 | 2 | ···· | 1 | 2 | 2 |
|  |  |  |  |  |  |  |  |  |
|  |  |  |  |  |  |  |  |  |
| 0231 | 3 | 3 | 3 | 3 | ···· | 3 | 3 | 3 |
| : | : | : | : | : | : | : | : | : |
| : | : | : | : | : | : | : | : | : |
| 0249 | 4 | 4 | 4 | 4 | ···· | 4 | 4 | 4 |
| 0250 | 2 | 3 | 2 | 3 | ···· | 2 | 2 | 2 |

CLASS DETERMINATION ~ S170

| ID | DETERMINED CLASS |
|---|---|
| 0001 | 1 |
| 0002 | 3 |
| 0003 | 2 |
| : | : |
| : | : |
| : | : |
| 0231 | 3 |
| : | : |
| : | : |
| 0249 | 4 |
| 0250 | 2 |

D170

## FIG. 10

NUMBER OF PEOPLE     MEDICAL EXPENSES

# FIG. 11

ID-CLASSIFIED DATA

| | | 2 0 1 4 | 2 0 1 5 | 2 0 1 6 | 2 0 1 7 |
|---|---|---|---|---|---|
| A HOSPITAL | MEDICAL TREATMENT INFORMATION STATEMENT | ◄——————————————————————————————► | | | |
| | NURSING CASE BENEFIT STATEMENT | ◄——————————————————————————————► | | | |
| | SPECIAL HEALTH CHECKUP RESULT | ◄——————————————————————————————► | | | |
| | INSURED MASTER | ◄——————————————————————————————► | | | |
| B HOSPITAL | MEDICAL TREATMENT INFORMATION STATEMENT | ◄——————————————————————————————► | | | |
| | NURSING CASE BENEFIT STATEMENT | ◄——————————————————————————————► | | | |
| | SPECIAL HEALTH CHECKUP RESULT | ◄——————————————————————————————► | | | |
| | INSURED MASTER | ◄——————————————————————————————► | | | |
| C HEALTH CHECKUP CENTER | MEDICAL TREATMENT INFORMATION STATEMENT | ◄——————————————————————► | | | |
| | NURSING CASE BENEFIT STATEMENT | ◄——————————————————————► | | | |
| | SPECIAL HEALTH CHECKUP RESULT | ◄——————————————————————► | | | |

:

:

| | | | | | |
|---|---|---|---|---|---|
| H MEDICAL OFFICE | NURSING CASE BENEFIT STATEMENT | ◄——————————————————————► | | | |
| | SPECIAL HEALTH CHECKUP RESULT | ◄——————————————————————————————► | | | |
| | INSURED MASTER | ◄——————————————————————► | | | |

# FIG. 12

(PRIOR VERIFICATION OF AI VALIDITY  PART 1)

(TEACHER PREDICTIVE DETERMINATION
DATA OF 250 CASES FOR YEAR 2015)

(ACTUAL DATA OF 50 CASES FOR YEAR 2014)

ID-CLASSIFIED FACTOR DATA

| PATIENT ID | EXPERT PREDICTION |
|---|---|
| 1 5 6 5 | 1 |
| 1 5 6 6 | 1 |
| 1 5 6 6 | 1 |
| 1 6 0 7 | 1 |
| ⋮ | |
| 2 1 2 0 | 3 |

AI PROCESS

(COMPARISON BETWEEN AI PREDICTION FOR
YEAR 2015 BASED ON DATA OF 50 CASES AND
ACTUAL DETERMINATION BY EXPERTS FOR
YEAR 2015)

| PATIENT ID | EXPERT ACTUAL DETERMINATION | AI PREDICTION |
|---|---|---|
| 1 5 6 5 | 1 | 1 |
| 1 5 6 6 | 1 | 1 |
| 1 5 6 6 | 1 | 1 |
| 1 6 0 7 | 1 | 2 |
| ⋮ | | |
| 2 1 2 0 | 3 | 3 |

# FIG. 13

(PRIOR VERIFICATION OF AI VALIDITY PART 2 (PREDICTION BASED ON 49 FACTOR ITEM DATA))

| AI SUPPLY DATA | | AI OUTPUT DATA | DATA FOR COMPARISON WITH AI OUTPUT |
|---|---|---|---|
| AI LEARNING DATA (CLASS PREDICTION DATA FOR YEAR 2015 BASED ON DATA FOR YEAR 2014 BY EXPERTS) | ACTUAL DATA FOR YEAR 2014 | CLASS PREDICTION DATA FOR YEAR 2015 | CLASS ACTUAL DETERMINATION DATA OF EXPERTS BASED ON ACTUAL DATA FOR YEAR 2015 |
| 2699 CASES | 6707 CASES | 6707 CASES | 6707 CASES |

(COMPARISON BETWEEN AI PREDICTION FOR YEAR 2015 AND EXPERT ACTUAL CLASS DETERMINATION DATA BASED ON ACTUAL DATA FOR YEAR 2015)

ACCURACY RATE

5 8. 9 0 %

## COMPARISON OF 6,707 CASES

| | AI DETERMINATION | EXPERT DETERMINATION |
|---|---|---|
| SEVERE LEVEL (4) | 281 | 48 |
| MIDDLE LEVEL (3) | 1199 | 1237 |
| LIGHT LEVEL (2) | 3054 | 3188 |
| HEALTH PROMOTION (1) | 2173 | 2234 |
| TOTAL | 6707 | 6707 |

EP 4 131 282 A1

# FIG. 14

(VERIFICATION OF EXPERT PREDICTIVE DETERMINATION)

MEDICAL WORKER PREDICTIVE DETERMINATION WAS RANDOMLY INPUTTED TO MULTIPLE AI PROCESSES, DEVIATION OF EXPERT PREDICTIVE DETERMINATION FOR NEXT YEAR BASED ON MULTIPLE AI OUTPUT WAS VERIFIED, AND EXPERT PREDICTIVE DETERMINATION DATA OF PREDETERMINED CONDITION WAS DELETED FROM TEACHER DATA

| PATIENT CODE | EXPERT PREDICTION | EACH AI EVALUATION | | | | | | | | | | AI STANDARD DEVIATION | DIFFER-ENCE |
|---|---|---|---|---|---|---|---|---|---|---|---|---|---|
| | | AI 1 | AI 2 | AI 3 | AI 4 | AI 5 | AI 6 | AI 7 | AI 8 | AI 9 | AI10 | | |
| 1000000000253 | 2 | 1 | 1 | 1 | 1 | 1 | 1 | 1 | 1 | 1 | 1 | 0 | 10 |
| 1000000000333 | 1 | 2 | 2 | 2 | 2 | 2 | 2 | 2 | 2 | 2 | 2 | 0 | 10 |
| 1000000000483 | 1 | 2 | 1 | 2 | 2 | 2 | 2 | 2 | 2 | 2 | 2 | 0.3 | 9 |
| 1000000000506 | 2 | 1 | 1 | 1 | 1 | 1 | 1 | 1 | 1 | 1 | 1 | 0 | 10 |
| 1000000000642 | 2 | 3 | 3 | 2 | 3 | 3 | 3 | 2 | 3 | 3 | 3 | 0.4 | 8 |
| 1000000000754 | 2 | 1 | 1 | 1 | 1 | 1 | 1 | 1 | 1 | 1 | 1 | 0 | 10 |
| 1000000000859 | 3 | 2 | 2 | 2 | 2 | 2 | 2 | 2 | 2 | 2 | 2 | 0 | 10 |
| 1000000000974 | 2 | 1 | 1 | 1 | 1 | 1 | 1 | 1 | 1 | 1 | 1 | 0 | 10 |
| 1000000001017 | 2 | 1 | 2 | 1 | 1 | 2 | 1 | 1 | 1 | 1 | 1 | 0.4 | 8 |
| 1000000001074 | 2 | 1 | 1 | 1 | 1 | 1 | 1 | 1 | 1 | 1 | 1 | 0 | 10 |
| 1000000001509 | 3 | 2 | 3 | 2 | 3 | 2 | 2 | 2 | 2 | 3 | 2 | 0.458 | 7 |
| 1000000001556 | 2 | 1 | 1 | 1 | 1 | 1 | 1 | 1 | 1 | 1 | 1 | 0 | 10 |
| 1000000001736 | 2 | 2 | 1 | 1 | 1 | 1 | 2 | 1 | 1 | 1 | 1 | 0.4 | 8 |
| 1000000001776 | 2 | 1 | 1 | 1 | 1 | 1 | 1 | 1 | 1 | 2 | 1 | 0.3 | 9 |
| 1000000001894 | 1 | 2 | 2 | 2 | 1 | 2 | 2 | 2 | 2 | 2 | 1 | 0.4 | 8 |
| 1000000001939 | 2 | 1 | 1 | 1 | 1 | 1 | 1 | 1 | 1 | 1 | 1 | 0 | 10 |
| ⋮ | | | | | | | | | | | | | |

EP 4 131 282 A1

# FIG. 15

EXPERT PREDICTIVE DETERMINATION DATA DETERMINED AS ABNORMAL VALUE AMONG 2699 CASES WAS ELIMINATED AND REMAINING 2228 CASES WERE USED AS TEACHER DATA

| AI SUPPLY DATA | | AI OUTPUT DATA | DATA FOR COMPARISON WITH AI OUTPUT |
|---|---|---|---|
| AI LEARNING DATA (TEACHER's CLASS DETERMINATION DATA AND PREDICTION FOR YEAR 2015 BASED ON DATA FOR YEAR 2014) | ACTUAL DATA FOR YEAR 2014 | CLASS PREDICTION DATA FOR YEAR 2015 | CLASS ACTUAL DETERMINATION DATA OF EXPERTS BASED ON ACTUAL DATA FOR YEAR 2015 |
| 2228 CASES | 6707 CASES | 6707 CASES | 6707 CASES |

REMARKABLE IMPROVEMENT OF CONSISTENCY RATE FOR ALL AI(1)-AI(10)

INDICATION OF AI PREDICTION SUITED TO PERSPECTIVE OF MEDICAL WORKER

## INTERNATIONAL SEARCH REPORT

| International application No. |
|---|
| PCT/JP2021/012705 |

| A.  CLASSIFICATION OF SUBJECT MATTER |
|---|
| G16H 50/20(2018.01)i; G06N 20/00(2019.01)i |
| FI: G06N20/00 130; G16H50/20 |
| According to International Patent Classification (IPC) or to both national classification and IPC |

| B.  FIELDS SEARCHED |
|---|
| Minimum documentation searched (classification system followed by classification symbols) |
| G16H50/20; G06N20/00 |

| Documentation searched other than minimum documentation to the extent that such documents are included in the fields searched | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

| Electronic data base consulted during the international search (name of data base and, where practicable, search terms used) |
|---|
| |

| C.  DOCUMENTS CONSIDERED TO BE RELEVANT | | |
|---|---|---|
| Category* | Citation of document, with indication, where appropriate, of the relevant passages | Relevant to claim No. |
| Y | US 2019/0362072 A1 (INTERNATIONAL BUSINESS MACHINES CORPORATION) 28 November 2019 (2019-11-28) paragraphs [0019], [0022], [0031], [0040]-[0043] | 1–14 |
| Y | JP 2013-148996 A (OSAKA UNIVERSITY) 01 August 2013 (2013-08-01) paragraphs [0050]-[0052], fig. 5A | 1–14 |
| Y | JP 2005-284348 A (SONY CORP.) 13 October 2005 (2005-10-13) paragraphs [0026]-[0030], [0092]-[0127] | 2-4, 6, 9-11, 13 |
| Y | JP 2005-209211 A (HONDA RESEARCH INSTITUTE EUROPE GMBH) 04 August 2005 (2005-08-04) paragraph [0070] | 3-4, 10-11 |
| Y | JP 2018-155522 A (SHIMADZU CORPORATION) 04 October 2018 (2018-10-04) paragraph [0032] | 3-4, 10-11 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 June 2021 (01.06.2021) | 08 June 2021 (08.06.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

| International application No. |
| --- |
| PCT/JP2021/012705 |

| Patent Documents referred in the Report | Publication Date | Patent Family | Publication Date |
| --- | --- | --- | --- |
| US 2019/0362072 A1 | 28 Nov. 2019 | (Family: none) | |
| JP 2013-148996 A | 01 Aug. 2013 | (Family: none) | |
| JP 2005-284348 A | 13 Oct. 2005 | US 2005/0220336 A1 paragraphs [0062]- [0066], [0132]-[0167] EP 1583024 A2 | |
| JP 2005-209211 A | 04 Aug. 2005 | US 2005/0209982 A1 paragraph [0085] EP 1557788 A2 | |
| JP 2018-155522 A | 04 Oct. 2018 | (Family: none) | |

Form PCT/ISA/210 (patent family annex) (January 2015)

**EP 4 131 282 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018173814 A
- JP 2019212146 A
- JP 2016206950 A